Europäisches Patentamt

European Patent Office

Office européen des brevets

(11) Publication number: **0 419 135 A2**

# EUROPEAN PATENT APPLICATION

(21) Application number: **90310041.0**

(51) Int. Cl.⁵: **A61K 31/52**

(22) Date of filing: **13.09.90**

| | |
|---|---|
| The title of the invention has been amended (Guidelines for Examination in the EPO, A-III, 7.3). | (71) Applicant: **BEECHAM-WUELFING GMBH** **Stresemannallee 6** **W-4040 Neuss(DE)** |
| (30) Priority: **16.09.89 GB 8921029** | (72) Inventor: **Jukna, Johannes** **Franz-Eger-Strasse 72** |
| (43) Date of publication of application: **27.03.91 Bulletin 91/13** | **W-3200 Hildesheim(DE)** |
| (84) Designated Contracting States: **AT BE CH DE DK ES FR GB GR IT LI LU NL SE** | (74) Representative: **Rutter, Keith et al** **Smith Kline Beecham, Corporate Patents,** **Great Burgh, Yew Tree Bottom Road** **Epsom Surrey KT18 5XQ(GB)** |

(54) **Use of 1,3-di-n-butyl-7-(2-oxopropyl)xanthine for treating disorders resulting from cerebral ischemia.**

(57) A method for the treatment of disorders resulting from a cerebral ischaemic event in mammals, which method comprises the administration of an effective, non-toxic, pharmaceutically acceptable amount of the compound, as defined, to the mammal in need thereof.

EP 0 419 135 A2

## NOVEL TREATMENT

The present invention relates to a method for the treatment of disorders resulting from a cerebral ischaemic event, especially functional disorders resulting from disturbed brain activity following the cerebral ischaemic event.

EP-A-0005015 discloses that 1,3-di-n-butyl-7-(2-oxopropyl xanthine ('the compound') is useful in the treatment of peripheral vascular disease. EP-A-0173039 discloses that certain xanthine and thioxanthine derivatives, including the compound, may be used for the treatment and/or prophylaxis of cerebrovascular disorders and/or disorders associated with cerebral senility.

Post-ischaemic patients generally require a prolonged period of rehabilitation following the effects of the ischaemic event. It has now surprisingly been indicated that treatment with the compound is of particular benefit to post-ischaemic patients.

In particular treatment with the compound is indicated to be of benefit for the treatment of functional disorders resulting from disturbed brain activity following the cerebral ischaemic event. This aspect of the invention relates to the treatment of disorders which fall within the general type of disorders disclosed in EP 0173039, but which are not specifically disclosed therein.

The compound may also have neuroprotectant activity. The compound may therefore be useful in the prophylaxis of disorders associated with neuronal degeneration resulting from cerebral ischaemic events.

Accordingly, the present invention provides a method for the treatment of disorders, especially functional disorders arising from disturbed brain activity, resulting from a cerebral ischaemic event in mammals, especially humans, which method comprises the administration of an effective, non-toxic, pharmaceutically acceptable amount of 1,3-di-n-butyl-7-(2-oxopropyl)xanthine to the mammal in need thereof.

Relevant cerebral ischaemic events include events caused by cardiac arrest and, especially, by stroke and also includes the cerebral ischaemia which may result from surgery. Also to be mentioned is the cerebral ischaemia which may occur in newborns during birth.

The disorders resulting from the cerebral ischaemic event include functional disorders resulting from disturbed brain activity following the cerebral ischaemic event, such as speech and locomotive disorders, sensory disorders, the loss of social skills. Also included are other such behavioural disorders associated with the post-ischaemic period.

The disorders resulting from the cerebral ischaemic event include disorders associated with neuronal degeneration following the ischaemic event.

The treatment of the invention encompasses both prophylactic treatment and treatment after the cerebral ischaemic event but in general, especially for the treatment of functional disorders, the treatment provides for administration of the compound after the cerebral ischaemic event. For the neuroprotectant treatment of disorders associated with neuronal degeneration, the treatment is preferably a prophylactic one.

The administration to the mammal may be by way of oral or parenteral administration.

An amount effective to treat the disorders hereinbefore described depends on the usual factors such as the nature and severity of the disorders being treated and the weight of the mammal. However, a unit dose will normally contain 1 to 1000 mg, suitably 1 to 500 mg, for example an amount in the range of from 2 to 100 mg such as 2, 5, 10, 20, 30, 40, 50 and 100 mg of the active compound. Unit doses will normally be administered once or more than once per day, for example 2, 3, 4, 5 or 6 times a day, more usually 2 to 4 times a day, such that the total daily dose is normally in the range, for a 70 kg adult of 1 to 1000 mg, for example 50 to 500 mg, that is in the range of approximately 0.01 to 15 mg/kg/day, more usually 0.1 to 10 mg/kg/day, for example 1 to 10 mg/kg/day.

It is greatly preferred that the compound is administered in the form of a unit-dose composition, such as a unit dose oral or parenteral composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p -hydroxybenzoate or sorbic acid, and if desired conventional flavouring or colouring agents.

Oral formulations also include conventional sustained release formulations, such as tablets or granules having an enteric coating.

For parenteral administration, fluid unit dose forms are prepared containing the compound and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

In addition such compositions may contain further active agents such as anti-hypertensive agents and diuretics.

As is common practice, the compositions will usually be accompanied by written or printed directions for use in the medical treatment concerned.

The present invention further provides a pharmaceutical composition for use in the treatment of disorders resulting from an ischaemic event, especially functional disorders arising from disturbed brain function, which comprises an effective, non-toxic, pharmaceutically acceptable amount of the compound and a pharmaceutically acceptable carrier therefor.

In a further aspect the invention provides the use of the compound for the manufacture of a medicament for the treatment of disorders resulting from an ischaemic event, especially functional disorders arising from disturbed brain activity.

Such compositions may be prepared in the manner as hereinbefore described.

The following pharmacological data illustrates the activity of the compound in tests which are indicative of the potential use of the compound for the treatment of the invention.

## PHARMACOLOGICAL DATA

## DELAYED NEURONAL DEATH AND FUNCTIONAL DEFECTS IN GERBILS AFTER TRANSIENT FOREBRAIN ISCHAEMIA

### Method

Ischaemia was produced in adult male gerbils by occlusion of both common carotid arteries for 3 minutes under halothane/nitrous oxide anaesthesia. During surgery the animals were placed upon a warming blanket. The ischaemia led to neuronal degeneration and to disturbed brain activity as measured by changes in functional behaviour.

Effect of the compound upon neuronal death

Brains were removed 2 weeks later and 12 μm thick coronal slices were examined light microscopically for neuronal degeneration.

Three groups of animals were used:

1. sham ligated controls
2. solvent-treated ligated controls
3. compound-treated ligated animals

The compound was administered 1 hour before the ligation period.

Results

Means ± SEM (n) of histopathological score [HPS] which reflects the degree of neuronal damage in the hippocampal CA1 field: 0 = no damaged neurons 1 = mild necrosis 2 = moderate necrosis 3 = severe necrosis 4 = complete necrosis

|  | Dose mg/kg | HPS | No of animals |
|---|---|---|---|
| sham ligated controls | - | 0 | (4) |
| ligated animals: |  |  |  |
| Solvent | - | 3.4 ± 0.3 | (21) |
| The compound | 5.0 i.p. | 1.5*± 0.5 | (14) |

*significantly (p ≤0.05) different from solvent treated ligated animals.

Conclusion

The compound significantly prevented ischaemia induced neuronal degeneration.

Effect of the compound on functional defects

Before and after the transient ischaemia animals were observed for behavioural changes reflecting ischaemia induced functional defects of the brain.

Results

After ischaemia the locomotor activity (running) of the ligated controls was significantly increased whilst stationary activity (nibbling) was decreased.

Animals treated with the compound (5mg/kg i.p.) given 1 hour after the ischaemia, did not develop functional defects.

4

| Time in seconds, means ± SEM, for the activities: | | | | |
|---|---|---|---|---|
| | RUNNING | | NIBBLING | |
| | pre- | post- | pre- | post- |
| | ligation | | ligation | |
| Controls (n = 9)<br>The compound 5mg/kg i.p. (n = 7) | 74 ± 9<br>70 ± 5 | 109* ± 11<br>68 ± 7 | 215 ± 46<br>185 ± 39 | 130* ± 36<br>198 ± 43 |

* significantly different from pre-value.

Conclusion

The compound normalized functional defects after an ischaemic period.

**Claims**

1. A use of 1,3-di-n-butyl-7-(2-oxopropyl)xanthine ('the compound') for the manufacture of a medicament for the treatment of disorders resulting from a cerebral ischaemic event in mammals.

2. A use according to claim 1, wherein the disorders are functional disorders arising from disturbed brain activity.

3. A use according to claim 1, wherein the disorders are selected from: speech and locomotive disorders, sensory disorders, loss of social skills and other behavioural disorders associated with the post-ischaemic period.

4. A use according to claim 1, wherein the compound is adapted for oral or parenteral administration.

5. A use according to claim 1, wherein the compound is administered in unit dosage form.

6. A use according to claim 5, wherein the unit dosage contains 1 to 1000mg of the compound.

7. A use according to claim 5, wherein the unit dosage contains 2 to 100mg of the compound.

8. A use according to claim 1, wherein the compound is administered to provide a total daily dosage in the range of 0.01 to 15mg/kg/day.